# EUROPEAN PATENT SPECIFICATION

(11) **EP 1 251 781 B1**
(45) Date of publication and mention of the grant of the patent: **06.12.2006**
(21) Application number: 01942528.9
(22) Date of filing: 23.01.2001
(51) Int. Cl.: A61B 10/00

(54) **DEVICE WITH MULTIPLE NEEDLES FOR OBTAINING SPECIMENS**
VORRICHTUNG MIT MEHREREN NADELN ZUR ENTNAHME VON PROBEN
DISPOSITIF A AIGUILLES MULTIPLES POUR LE PRELEVEMENT D'ECHANTILLONS

(30) Priority: 24.01.2000 SE 0000227; 25.01.2000 SE 0000245; 28.01.2000 SE 0000292; 01.12.2000 SE 0004451
(43) Date of publication of application: 30.10.2002
(73) Proprietor: AB Christer Dahlstrand, 411 36 Göteborg (SE)
(72) Inventor: DAHLSTRAND, Christer, S-413 19 Göteborg (SE)
(74) Representative: Cederbom, Hans Erik August
(86) International application number: PCT/SE2001/000114
(87) International publication number: WO 2001/052742

(56) References cited:
- US-A- 5 415 182

## Description

The present invention relates to an arrangement enabling a number of biopsies to be obtained in a single sampling insertion with needles connected in such a way as to move together at one time.

The present invention provides an improved method of obtaining tissue for examination, for example under the microscope, for monitoring purposes and for establishing or excluding the presence of disease. The invention permits several biopsies to be taken at the same time in a predetermined geometrical pattern.

When examining tissue for the presence of suspected disease, for monitoring purposes or for some other reason, a number of methods can be used, for example x-ray, ultrasound, blood sampling and other techniques. It is very common for a tissue sample to be required, known as a biopsy. This is particular true if there may be a possibility of a malignant disease, i.e. cancer. The sample is usually taken using some form of needle, which is hollow so that a core of tissue is obtained. The needle can be inserted manually or with the help of various instruments, for example spring-loaded so-called biopsy pistols. The needle is aimed manually or with the help of ultrasound, x-ray or some other equipment. It is usual to take a number of biopsies. Too many biopsies cannot be taken for a variety of reasons, mainly because every insertion is painful for the patient. One organ that is often examined is the prostate, from which it usual to take six biopsies. Unfortunately, this means that the proportion of the organ that is examined is small. For example, six biopsies of 1.2 mm in diameter and 20 mm in length are usually taken from the prostate. The gland itself has a volume of perhaps approximately 50 cubic centimetres, and in actual fact the samples obtained accordingly represent only a few percent of the volume of the gland. It is also true, in many cases, that less significance is attached to changes below a certain size. These are considered to be so small that they do not warrant any intervention, for example in the form of an operation, but can continue to be monitored.

Previously disclosed in US 5,415,182 A is a biopsy instrument equipped with a number of needles, although in this case all the needles are arranged in parallel and in such a way as to move in separate paths at a mutual lateral distance from one another, viewed across the longitudinal extent and in the direction of travel of the needles. The preamble of claim 1 is based on the disclosure of US-A-5 415 182.
Problems are also encountered with instruments of the above kind, since sampling takes place in the same direction with all the needles, and the area examined is consequently small and narrow. The instrument in this case is also unwieldy and cannot be aimed with the help of ultrasound, for example.

### Object of the invention

The principal object of the present invention is thus, in the first instance, to solve the aforementioned problems by simple and effective means.

### Most important characteristics of the invention

The aforementioned object is achieved by means of an arrangement as defined in claim 1.

### Drawings

The invention is described below as a number of preferred illustrative embodiments, in conjunction with which reference is made to the accompanying drawings, in which:
Fig. 1 shows a perspective view of an example of an arrangement in accordance with invention viewed at an angle from the front;
Fig. 2 shows the front part of the arrangement;
Fig. 3 shows a sectioned view along the line III-III in Fig. 2 and with needle extensions in a three-legged pattern;
Fig. 4 shows a further example of a needle extension in the curved form of a fan; but forms no part of the invention,
Fig. 5 shows examples of sampling insertions in a prostate gland using the invention; and
Fig. 6 shows the pattern of holes respectively for the prior art and for the invention.

The invention draws on the principle partly that a greater number of samples is obtained more rapidly if several tissue samples are taken simultaneously, and partly that fewer insertions are required by using double or triple or multiple needles. The human body is unable to distinguish whether one or more insertions are being made if they lie close together, for example separated by three millimetres, and if the insertions take place simultaneously. This means that a double or triple insertion will cause no more pain than a single insertion, and four triple insertions = twelve biopsies will feel like four individual insertions that would produce only four biopsies.

A volume effect is also gained, since three biopsies are taken in a fixed form, for example in a triangle. A certain volume of tissue is required in order to be able to determine whether tumours or changes are present there. The volume available for determination can be increased tenfold or more in this way. I.e. if the needles are arranged 3 mm apart in the form of a triangle, a specific volume will be obtained, from which it is possible to determine with certainty that no changes of 3 mm in size are present.

The arrangement can also consist of a number of tubes or channels, for example in an ultrasound probe, which guide the biopsy needles so that they form the desired geometrical pattern in the tissue from which the samples are to be taken.

An arrangement 1 to enable a number cf biopsies to be obtained in a single sampling insertion with needles 2-4 connected in such a way as to move together at one time in accordance with the present invention has at least one 2 of its two or more needles 2-4 so arranged as to extend in a specific path 5 from the paths 6, 7 of other needles 3, 4. This permits several biopsies of the intended tissue 8 to be taken at the same time in an established geometrical pattern.

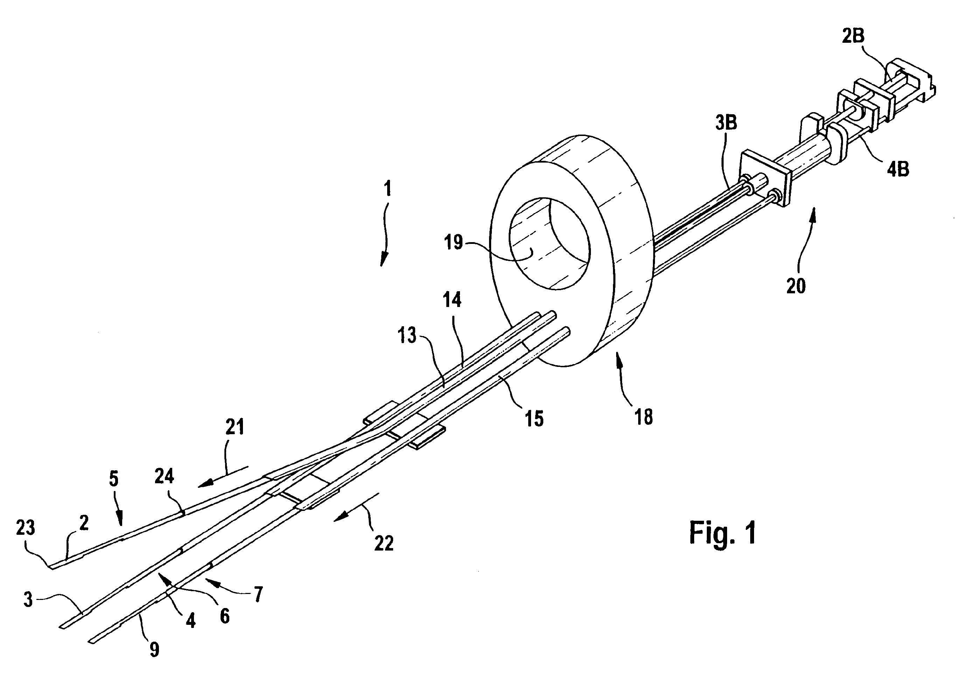

Illustrated in the drawings are two different embodiments of examples of the extent of needles in accordance with the invention, and in accordance with a first example shown in the drawings in Figs. 1-3, two of the needles 3, 4 extend essentially parallel with one another, while an angled needle 2 situated between the two parallel needles 3, 4 is so arranged as to extend along a curved path 5. The aforementioned curved path 5 is separate from the two essentially straight paths 6, 7 for the two straight needles 3, 4.

The needles 2-4 can be of a previously disclosed kind, which exhibits a both radial and axial elongated depression 9 at their front end 2A-4A, and which consists of spring steel, for example. A needle of this kind is referred to in the medical profession as a mandrin. The needle, which preferably exhibits a circular cross-section, is so arranged as to be accommodated in and guided by a cylindrical inner sleeve 10, 11, 12, each of which in turn is so accommodated as to be capable of displacement inside its own cylindrical outer sleeve 13, 14, 15.

Alternatively, the needles 2-4 can be caused to be guided by a number of matching holes or channels in the actual instrument. I.e, the aforementioned sleeves are replaced by similar holes or channels.

In accordance with the first example, the three needles 2-4 are uniformly distributed when viewed around the periphery of the needle group.

Naturally, the number of needles can vary. The smallest number of needles can be from two upwards to an unlimited number. The principal requirement is for the needles to be separated laterally by a certain distance from one another, although not to such an extent that the common insertion which all the needles are intended to give at the same time in one and the same introduction into the body are perceived by the patient as more than one insertion at the same time.

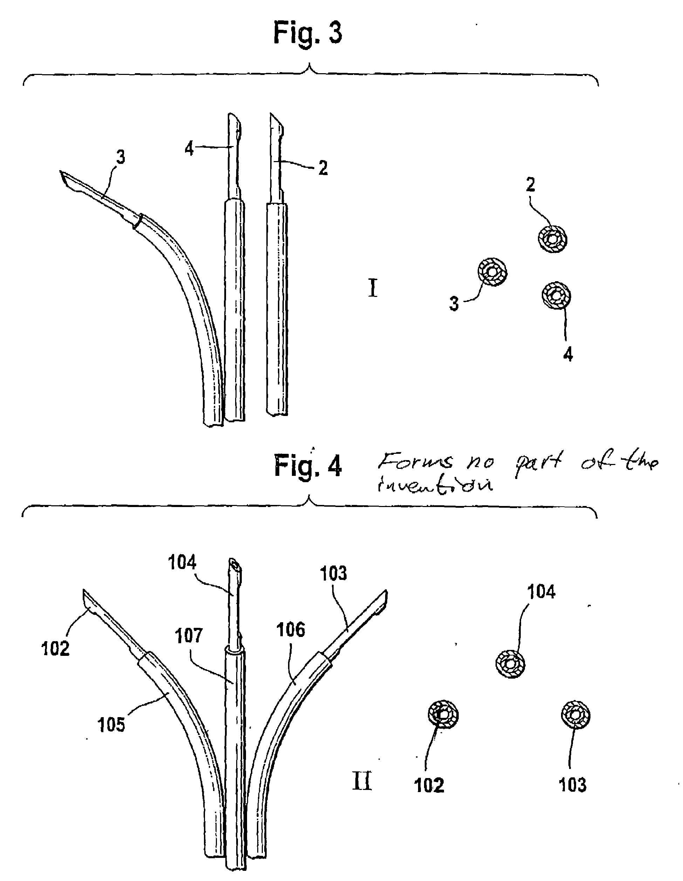

In accordance with a second illustrative embodiment II of needles shown in the drawings in Fig. 4, each of at least two needles 102, 103 of three needles 102, 103, 104 extends in its own curved track 105, 106, although preferably the third needle 104 also extends along a curved track 107 in the form of a fan, but which forms no part of the invention.

The needles in the intended examples are thus so arranged as to extend in the form of a prism.

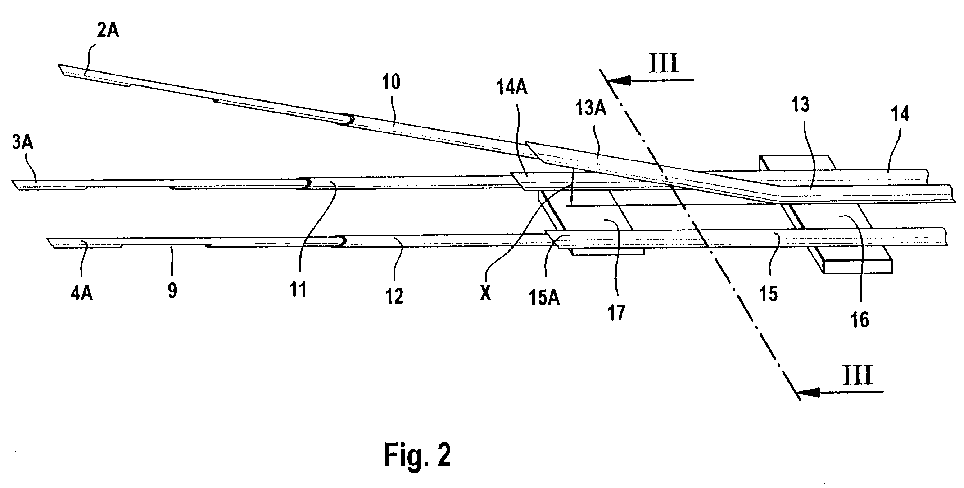

The aforementioned outer sleeves 13-15 are arranged in fixed positions by being attached to a common inner guide sleeve 16, for example in the form of a platform-like component to which the aforementioned outer sleeves 13-15 are attached by soldering, for example as shown in Fig. 2, at a distance from the free outer ends 13A-15A of the aforementioned outer sleeves 13-15. An outer sleeve 13 extending in this way at a desired angle x with a straight form or in a curved path is so arranged as to extend freely at an angle x from an outer guide holder 17, which holds together the two upper straight outer sleeves 14, 15 at their respective free outer ends 14A, 15A.

The drawings illustrate how the invention can be applied to sampling the prostate gland 8, in conjunction with which a common holder 18 is utilized for the needles 2-4 and the sleeves 10-12 and 13-15. The aforementioned common holder is formed from a jig which exhibits a central opening 19 for the attachment of the jig to a transmitter and receiver (not shown) which function with ultrasound. The needles 2-4 are connected for this purpose with their respective rear parts 2B-4B to a control arrangement 20, which is so arranged in a previously disclosed fashion as to be inserted in a pistol, for example of the kind shown in the aforementioned US 5,415,182 A. In an initial stage, the needles 2-4 are first caused to be displaced in a direction 21 outwards from the associated inner sleeve 10-12, and the inner sleeves are then caused to be displaced outwards in the direction 22, so that the needles are enclosed by them. Everything is effected by the application of spring force and takes place so quickly that it is perceived as a common needle insertion. The common withdrawal of needles 2-4 and inner sleeves 10-12 then takes place in the same operation. A tissue sample will now have been removed from the intended sampling location with the help of the respective depression 9 in the respective needle and the enclosing cutting inner tube 10-12. The needles 2-4 and inner tubes 10-12 each exhibit a tip 23, 24 such that they penetrate easily into the test object 8 and take the desired tissue samples.

The invention permits the application of a sampling method for tissue sampling, in which the volume of tissue required for examination in respect of changes of a certain size is obtained by inserting two or more needles in a predetermined geometrical pattern, whereby the pain for the patient is reduced by inserting several biopsy needles at the same time at a distance from one another such that the body perceives this as a single insertion.

Two or more biopsy needles are guided in a predetermined geometrical pattern by a number of tubes or channels, into which the biopsy needles are inserted simultaneously and are guided thereby into the tissue to be examined.

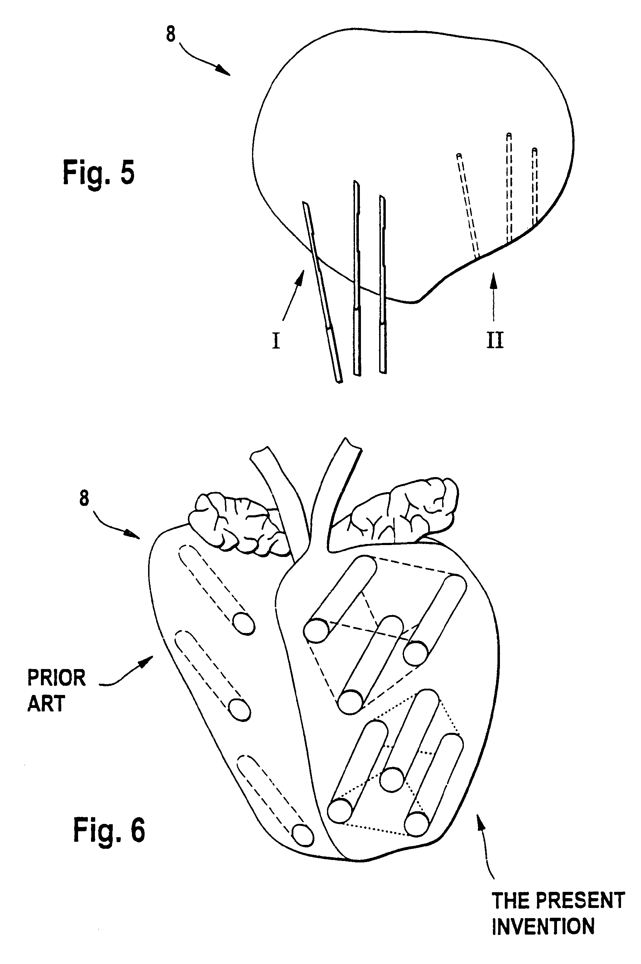

The insertion pattern for the two sets of needles I and II is shown in Fig. 5, which means that, if the two sets of needles are used, it is easy to investigate a desired organ and take samples such that, on the one hand, it is possible to investigate the whole of the organ in question with a small number of common needle insertions and, in addition, to examine the extent of any tumour, etc., that may be found.

The left side of the drawing in Fig. 6 shows the nature of the pattern of holes used with the prior art, while the right side of the drawing shows examples of hole patterns in accordance with the present invention.

Whereas, with single needles, one was previously obliged to insert the needle six times, and this procedure very often had to be repeated at several different consultations in order to be certain of the test results, it is now often sufficient to make four insertions, each with three needles, in the periphery of the prostate. The number of needles taking samples has thus been increased effectively from six up to as many as twelve, with fewer insertions.

Guiding of the needles takes place with the help of a visual display screen on which the needles can be seen inside the body of the patient after the transmitter/receiver has been inserted through the patient's rectum as far as the intended test object, for example a prostate gland 8. It is possible in this case to display an image on the screen with an inserted target line, and it is then possible to push in different directions and with different patterns depending on the sets of needles contained in the apparatus. At least two needles, but preferably four, are thus used simultaneously.

The volume of the gland that is examined may now be significantly greater than for investigation using the prior art. It is possible to state with greater certainty that the entire object has been examined. The possibility is now also available at the time of dioptry for reaching a conclusion about the size of any tumour that may be present.

The needles in question are curved or are formed in some other way, such that they extend and together form a geometrical figure, which, in conjunction with repeated penetration of the tissue in question, is so arranged as to provide high coverage of the mass and configuration of the organ under investigation.

For example, either all the needles can be curved so that each extends essentially in parallel with the others along its own curved path, or all the needles can be so arranged that each extends along its own double-curved path but at a set distance from the others, for example they are formed so that they extend along at least a part of a sphere, for example resembling the form of the curved head part of a spoon.

A further example of the extent of the needles is that they diverge or converge in relation to one another. If only two needles are present, the investigated test volume will only be a narrow column, but if the number of needles present is more than two, the investigated test volume will have the intended desired configuration, e.g. in the form of a cone or a pyramid, etc.

It is also possible to provide a configuration and a multiplicity of needles which are so arranged, with the help of a mathematical model, as to permit the assessment of the size of a tumour that is being looked for on the basis of the number of hits on the aforementioned tumour.

It is also possible to connect the instrument to the ultrasound probe, for example, with the help of a number of channels drilled or milled in the actual probe.

The invention is naturally not restricted to the embodiments described above and illustrated in the accompanying drawings. Modifications are possible, in particular in relation to the nature of the various component parts, or by the use of equivalent technology, without departing from the protected area of the invention as it is defined in the Patent Claims.

## Claims

1. Arrangement (1) to enable a number of biopsies to be obtained in a single sampling insertion with needles (2-4; 102-104) connected in such a way as to move together at one time, in which at least three needles are present, ***characterized in that*** three needles (2-4; 102-104) are arranged in the form of a triangle **in that** at least one (2) of the needles (2-4; 102-104) is so arranged as to extend in a separate path (5) from the other needle/needles (3-4), **in that** two of the needles (3, 4) extend essentially parallel with one another with an angled needle (2) situated between the two parallel needles (3, 4), which enables a number of biopsies of the intended tissue (8) to be taken simultaneously in a predetermined geometrical pattern.

2. Arrangement in accordance with Patent Claim 1, ***characterized in that*** the third needle (2) extends along a curved path (5).

3. Arrangement in accordance with Patent Claim 2, ***characterized in that*** the three needles (2-4) are uniformly distributed when viewed around the periphery of the needle group.

4. Arrangement in accordance with one or other of Patent Claims 1-3, ***characterized in that*** at least two of the needles (102-104) are curved.

5. Arrangement in accordance with one or other of Patent Claims 1-4, ***characterized in that*** each of the needles (2-4; 102-104), which are in the form of a mandrin with a depression (9) for sampling, is accommodated in and guided by a cylindrical inner sleeve (10-12) capable of axial displacement in a fixed outer sleeve (13-15).

6. Arrangement in accordance with Patent Claim 5, ***characterized in that*** all the outer sleeves (13-15) are attached to a common inner guide holder (16) at a distance from their free outer ends (13A-15A), and **in that** an angled outer sleeve (13) extends freely at an angle (x) from an outer guide holder (17).

7. Arrangement in accordance with one or other of Patent Claims 5-6, ***characterized in that*** the outer sleeves (13-15) are accommodated so that they are held in a position relative to one another by a common holder (16).

8. Arrangement in accordance with Patent Claim 7, ***characterised in that*** the holder is formed from a jig (18), which exhibits a central opening (19) for the attachment of the jig (18) to a transmitter and receiver.

9. Arrangement in accordance with one or other of Patent Claims 1-8, ***characterized in that*** the needles in question are'curved or are formed in some other way such that they extend and together form a geometrical figure, which, in conjunction with repeated penetration of the tissue in question, is so arranged as to provide high coverage of the mass and configuration of the organ under investigation.

10. Arrangement in accordance with one or other of Patent Claims 1-9, ***characterized in that*** a configuration and a multiplicity of needles is so arranged, with the help of a mathematical model, as to permit the assessment of the size of a tumour that is being looked for on the basis of the number of hits on the aforementioned tumour.

## Patentansprüche

1. Anordnung (1), um zu ermöglichen, eine Anzahl von Biopsien in einem einzigen Probenentnahmeeinsatz mit Nadeln (2-4; 102-104) zu erhalten, die so verbunden sind, dass sie sich gleichzeitig miteinander bewegen, bei der mindestens drei Nadeln vorhanden sind,
**dadurch gekennzeichnet, dass**
drei Nadeln (2-4; 102-104) in Form eines Dreiecks angeordnet sind, in dem mindestens eine (2) der Nadeln (2-4; 102-104) so angeordnet ist, dass sie sich auf einem separaten Weg (5) von der anderen Nadel/den anderen Nadeln (3-4) weg erstreckt, dass sich zwei der Nadeln (3, 4) im Wesentlichen parallel zueinander erstrecken, wobei eine abgewinkelte Nadel (2) zwischen den beiden parallelen Nadeln (3, 4) angeordnet ist, was ermöglicht, eine Anzahl von Biopsien des betrachteten Gewebes (8) in einem vorbestimmten geometrischen Muster gleichzeitig zu entnehmen.

2. Anordnung nach Anspruch 1, **dadurch gekennzeichnet, dass** sich die dritte Nadel (2) entlang eines gekrümmten Wegs (5) erstreckt.

3. Anordnung nach Anspruch 2, **dadurch gekennzeichnet, dass** die drei Nadeln (2-4) aus der Sicht um den Umfang der Nadelgruppe gesehen gleichmäßig verteilt sind.

4. Anordnung nach einem der Ansprüche 1 - 3, **dadurch gekennzeichnet, dass** mindestens zwei der Nadeln (102-104) gekrümmt sind.

5. Anordnung nach einem der Ansprüche 1 - 4, **dadurch gekennzeichnet, dass** sich jede der Nadeln (2-4; 102-104), welche die Form eines Mandrins mit einer Vertiefung (9) für eine Probenentnahme aufweisen, in einer zylinderförmigen inneren Hülse (10-12), die zu einer axialen Verschiebung in einer festen äußeren Hülse (13-15) fähig ist, befindet und durch diese geführt ist.

6. Anordnung nach Anspruch 5, **dadurch gekennzeichnet, dass** alle äußeren Hülsen (13-15) in einer Entfernung von ihren freien äußeren Enden (13A-15A) an einem gemeinsamen inneren Führungshalter (16) angebracht sind, und dass sich eine abgewinkelte äußere Hülse (13) frei unter einem Winkel (x) von einem äußeren Führungshalter (17) erstreckt.

7. Anordnung nach Anspruch 5 oder 6, **dadurch gekennzeichnet, dass** die äußeren Hülsen (13-15) so angeordnet sind, dass sie durch einen gemeinsamen Halter (16) in einer Position relativ zueinander gehalten sind.

8. Anordnung nach Anspruch 7, **dadurch gekennzeichnet, dass** der Halter aus einer Spannvorrichtung (1.8) gebildet ist, die eine zentrale Öffnung (19) für die Anbringung der Spannvorrichtung (18) an einem Sender und einem Empfänger aufweist.

9. Anordnung nach einem der Ansprüche 1-8, **dadurch gekennzeichnet, dass** die betreffenden Nadeln gekrümmt sind oder auf eine andere Weise so ausgebildet sind, dass sie sich erstrecken und miteinander eine geometrische Figur bilden, die in Verbindung mit einer wiederholten Penetration des betreffenden Gewebes so angeordnet ist, dass sie eine hohe Abdeckung der Masse und der Struktur des untersuchten Organs bereitstellt.

10. Anordnung nach einem der Ansprüche 1-9, **dadurch gekennzeichnet, dass** eine Ausgestaltung und eine Vielzahl von Nadeln so angeordnet ist, dass mit Hilfe eines mathematischen Modells die Abschätzung der Größe eines gesuchten Tumors auf der Grundlage der Anzahl von Treffern an dem zuvor genannten Tumor möglich ist.

## Revendications

1. Dispositif (1) permettant de réaliser plusieurs biopsies en une seule insertion de prélèvement d'échantillons par aiguilles (2 à 4 ; 102 à 104) raccordées de manière à se déplacer ensemble à la fois, dans lequel au moins trois aiguilles sont présentes, **caractérisé en ce que** les trois aiguilles (2 à 4 ; 102 à 104) sont disposées en triangle, **en ce qu'**au moins une aiguille (2) parmi les aiguilles (2 à 4 ; 102 à 104) est prévue de manière à se prolonger selon une voie différente de l'autre ou des autres aguille(s) (3 à 4), et **en ce que** deux des aiguilles (3, 4) se prolongent sensiblement parallèles l'aiguille (2) située entre les deux aiguilles parallèles (3, 4) étant inclinée, ce qui permet de réaliser un certain nombre de biopsies du tissu (8) prévu pour être prélevé selon une disposition géométrique prédéterminée.

2. Dispositif selon la revendication 1, **caractérisé en ce que** la troisième aiguille (2) s'étend selon une courbe (5).

3. Dispositif selon la revendication 2, **caractérisé en ce que** les trois aiguilles (2 à 4) sont réparties de manière uniforme lorsque observées du pourtour du groupe d'aiguilles.

4. Dispositif selon l'une quelconque des revendications 1 à 3, **caractérisé en ce qu'**au moins deux des aiguilles (102 à 104) sont courbes.

5. Dispositif selon l'une quelconque des revendications 1 à 4, **caractérisé en ce que** chacune des aiguilles (2 à 4 ; 102 à 104), qui se présente sous la forme d'un mandrin avec un évidement pour le prélèvement d'échantillons, est logée dans et guidée par une gaine interne cylindrique (10 à 12) pouvant se déplacer de manière axiale dans un fourreau externe fixe (13 à 15).

6. Dispositif selon la revendication 5, **caractérisé en ce que** tous les fourreaux externes (13 à 15) sont fixés à un support commun de guidage interne (16) située à distance de leurs extrémités libres externes (13A à 15A), et **en ce qu'**un fourreau externe incliné (13) se prolonge librement selon un angle (x) à partir d'un support de guidage externe (17).

7. Dispositif selon l'une quelconque des revendications 5 à 6, **caractérisé en ce que** les fourreaux externes (13 à 15) sont prévus de manière à pouvoir être maintenus en place les uns par rapport aux autres par un support commun (16).

8. Dispositif selon la revendication 7, **caractérisé en ce que** le support est formé à partir d'une monture (18) présentant une ouverture centrale (19) pour la fixation de la monture (18) à un émetteur-récepteur.

9. Dispositif selon l'une quelconque des revendications 1 à 8, **caractérisé en ce que** les aiguilles en question sont courbes ou sont conformées autrement de sorte qu'elles se prolongent et forment ensemble une figure géométrique qui, en combinaison avec la pénétration répétée dans le tissu en question, est prévue de manière à fournir une large couverture de la masse et de la conformation de l'organe examiné.

10. Dispositif selon l'une quelconque des revendications 1 à 9, **caractérisé en ce qu'**une configuration et une multiplicité d'aiguilles sont prévues, à l'aide d'un modèle mathématique, de manière à permettre l'estimation de la taille d'une tumeur qui est recherchée en se basant sur le nombre de coups portés sur la tumeur susmentionnée.
